# EUROPEAN PATENT APPLICATION

(11) **EP 1 233 075 A2**
(43) Date of publication of application: **21.08.2002**
(21) Application number: 02251007.7
(22) Date of filing: 14.02.2002
(51) Int. Cl.: C12Q 1/68

(54) **BDNF polymorphism and association with bipolar disorder**

(30) Priority: 15.02.2001 US 269059 P
(71) Applicant: WHITEHEAD INSTITUTE FOR BIOMEDICAL RESEARCH, Cambridge, MA 02142 (US); THE GENERAL HOSPITAL CORPORATION, Boston, MA 02114 (US); JOHNS HOPKINS UNIVERSITY, Baltimore, MD 21205 (US)
(72) Inventor: Sklar, Pamela, Brookline, Massachusetts 02446 (US); Lander, Eric S., Cambridge, Massachussetts 02139 (US); Depaulo, Raymond J., Jr., Baltimore, Massachusetts 21204-6526 (US); McInnis, Melvin G., Timonium, Maryland 21093 (US)
(74) Representative: Kirkham, Nicholas Andrew

(57) **Abstract**

Methods for diagnosing and treating neuropsychiatric disorders, especially bipolar disorder, and to methods for identifying compounds for use in the diagnosis and treatment of neuropsychiatric disorders are disclosed. Also disclosed are novel compounds and pharmaceutical compositions for use in the diagnosis and treatment of neuropsychiatric disorders such as bipolar disorder.

SEQUENCE LISTING

<110> Sklar, Pamela
      Lander, Eric S.
      DePaulo, J. Raymond, Jr.
      McInnis, Melvin G.

<120> BDNF Polymorphism and Association with
      Bipolar Disorder

<130>

<150> US 60/269,059
<151> 2001-02-15

<160> 5

<170> FastSEQ for Windows Version 4.0

<210> 1
<211> 1685
<212> DNA
<213> Homo sapiens

<400> 1
tgtaaaacag gatggctcaa tgaaattatc tttctttctt ctataataga gtatctctgt 60
gggaagagga aaaaaaaagt caatttaaag gctccttata gttccccaac tgctgtttta 120
ttgtgctatt catgcctaga catcacatag ctagaaaggc ccatcagacc cctcaggcca 180
ctgctgttcc tgtcacacat tcctgcaaag gaccatgttg ctaacttgaa aaaaattact 240
attaattaca cttgcagttg ttgcttagta acatttatga ttttgtgttt ctcgtgacag 300
catgagcaga gatcattaaa aattaaactt acaaagctgc taaagtggga agaaggagaa 360
cttgaagcca caatttttgc acttgcttag aagccatcta atctcaggtt atatgctaga 420
tcttgggggc aaacactgca tgtctctggt ttatattaaa ccacatacag cacactactg 480
acactgattt gtgtctggtg cagctggagt tatcaccaag acataaaaaa accttgaccc 540
tgcagaatgg cctggaatac aatcagaggg ccacatggca tcccggtgaa agaaagccct 600
aaccagtttt ctgtcttgtt tctgctttct ccctacagtt ccaccaggtg agaagagtga 660
tgaccatcct tttccttact atggttattt catactttgg ttgcatgaag gctgccccca 720
tgaaagaagc aaacatccga ggacaaggtg gcttggccta cccaggtgtg cggacccatg 780
ggactctgga gagcgtgaat gggcccaagg caggttcaag aggcttgaca tcattggctg 840
acactttcga acacatgata gaagagctgt tggatgagga ccagaaagtt cggcccaatg 900
aagaaaacaa taaggacgca gacttgtaca cgtccagggt gatgctcagt agtcaagtgc 960
ctttggagcc tcctcttctc tttctgctgg aggaatacaa aaattaccta gatgctgcaa 1020
acatgtccat gagggtccgg cgccactctg accctgcccg ccgaggggag ctgagcgtgt 1080
gtgacagtat tagtgagtgg gtaacggcgg cagacaaaaa gactgcagtg gacatgtcgg 1140
gcgggacggt cacagtcctt gaaaaggtcc ctgtatcaaa aggccaactg aagcaatact 1200
tctacgagac caagtgcaat cccatgggtt acacaaaaga aggctgcagg ggcatagaca 1260
aaaggcattg gaactcccag tgccgaacta cccagtcgta cgtgcgggcc cttaccatgg 1320
atagcaaaaa gagaattggc tggcgattca taaggataga cacttcttgt gtatgtacat 1380
tgaccattaa aaggggaaga tagtggattt atgttgtata gattagatta tattgagaca 1440
aaaattatct atttgtatat atacataaca gggtaaatta ttcagttaag aaaaaaataa 1500
ttttatgaac tgcatgtata aatgaagttt atacagtaca gtggttctac aatctattta 1560
ttggacatgt ccatgaccag aagggaaaca gtcatttgcg cacaacttaa aaagtctgca 1620
ttacattcct tgataatgtt gtggtttgtt gccgttgcca agaactgaaa acataaaaag 1680
ttaaa                                                             1685

<210> 2
<211> 247
<212> PRT
<213> Homo sapiens

<400> 2

<210> 3
<211> 19
<212> DNA
<213> Artificial Sequence

<220>
<223> FRET Primer

<400> 3
ggctgacact ttcgaacac                                                19

<210> 4
<211> 40
<212> DNA
<213> Artificial Sequence

<220>
<223> Primer

<400> 4
tgtaaaacga cggccagtct tgacatcatt ggctgacact                         40

<210> 5
<211> 45
<212> DNA
<213> Artificial Sequence

<220>
<223> Primer

<400> 5
taatacgact cactataggg gtacaagtct gcgtccttat tgttt                45

## Description

Modern psychiatry typically subdivides mood disorders into bipolar disorders (episodes of mania or both mania and depression) and unipolar depressive disorder (episodes of depression). Symptoms of mania include expansive, elevated or irritable mood, inflated self-esteem, grandiosity, decreased need for sleep, increased talkativeness, racing thoughts, distractibility, increased goal-directed activity, and excessive involvement in pleasurable activities with a high potential for painful consequences. Depressive symptoms include depressed mood, diminished interest or pleasure in activities, insomnia or hypersomnia, psychomotor agitation or retardation, fatigue or loss of energy, feelings of worthlessness, excessive guilt, inability to concentrate or act decisively, and recurrent thoughts of death or suicide. Several mental disorders have been proposed as alternate expressions of a bipolar genotype, including variants of schizoaffective disorder, recurrent unipolar depression and hypomania (bipolar II disorder).

Neuropsychiatric disorders, such as schizophrenia, attention deficit disorders, schizoaffective disorders, bipolar disorders and unipolar disorders, differ from neurological disorders in that anatomical or biochemical pathologies arc readily detectable for the latter but not the former. Largely as a result of this difference, drugs which have been used to treat individuals with neuropsychiatric disorders, including lithium salts, valproic acid and carbamazepine, have not been predictably effective in treatment regimens across a variety of patients. Treatment regimens are further complicated by the fact that clinical diagnosis currently relics on clinical observation and subjective reports. Identificalion of the anatomical or biochemical defects which result in neuropsychiatric disorders is needed in order to effectively distinguish between the disorders and to allow the design and administration of effective therapeutics for these disorders,

As described herein, polymorphisms in the gene for brain-derived neurotrophic factor (BDNF) have been discovered, and at least one of the polymorphisms is correlated with incidence of neuropsychiatric disorders (e.g., bipolar disorder). A polymorphism at nucleotide position 31 in human brain-derived neurotrophic factor (as numbered in SEQ ID NO: 1, GenBank Accession No: M61181) has been discovered in which the reference "T" (thymine) is changed to "A" (adenine).

Furthermore, a single nucleotide polymorphism has been discovered within the nucleotide sequence encoding the 128 amino acid prepro portion of the BDNF gene product which is correlated with reduced incidence of bipolar disorder in a sample population assessed as described herein. In one embodiment, a single nucleotide polymorphism from "G" to "A" at nucleotide position 858 (as numbered in SEQ ID NO: 1), resulting in an amino acid change from valine to methionine at amino acid position -63 (relative to the start of the mature protein), is correlated with a reduced incidence of bipolar disorder in the sample population assessed as described herein. That is, it has been determined that there is a variation from random (i.e., that which would be expected by chance) in the transmission of the reference "G" (guanine) and variant "A" (adenine) at position 858 from a parent who is heterozygous for the BDNF alleles to an offspring diagnosed with bipolar disorder. It appears that this variant allele of the SNP in the prepro region of BDNF may contribute to protection or reduction in symptomology with respect to bipolar disorder. Alternatively, this particular polymorphism may be one of a group of two or more polymorphisms in the BDNF gene which contributes to the presence, absence or severity of the neuropsychiatric disorder, e.g., bipolar disorder.

The invention relates to methods for diagnosing and treating neuropsychiatric disorders, especially bipolar disorder, and to methods for identifying compounds for use in the diagnosis and treatment of neuropsychiatric disorders. The invention relates to novel compounds and pharmaceutical compositions for use in the diagnosis and treatment of neuropsychiatric disorders. The invention further relates to kits for use in diagnosing neuropsychiatric disorders. In a preferred embodiment, the neuropsychiatric disorder is bipolar disorder.

In one embodiment, the invention relates to a method for predicting the likelihood that an individual will have a neuropsychiatric disorder (or aiding in the diagnosis of a neuropsychiatric disorder), e.g., bipolar disorder, comprising the steps of obtaining a DNA sample from an individual to be assessed and determining the nucleotide present at nucleotide position 31 of the BDNF gene, as numbered in SEQ ID NO: 1. The presence of a "T" at position 31 indicates that the individual has a reduced likelihood of being diagnosed with a neuropsychiatric disorder than an individual having an "A" at that position. In a preferred embodiment, the neuropsychiatric disorder is bipolar disorder. In a particular embodiment, the individual is an individual at risk for development of bipolar disorder.

The method comprises obtaining a DNA sample from an individual to be assessed. The DNA sample comprises a polynucleotide sequence of the BDNF gene or portion thereof comprising position 31 of SEQ ID NO: 1. The nucleotide present at position 31 of said polynucleotide sequence is determined. The identity of the nucleotide at position 31 can be determined by nucleic acid detection methods well known in the art.

In another embodiment, the invention is drawn to a method of predicting the likelihood that an individual will have reduced symptomology associated with a neuropsychiatric disorder, comprising the steps of obtaining a DNA sample from an individual to be assessed and determining the nucleotide present at nucleotide position 31 of the BDNF gene, as numbered in SEQ ID NO: 1. The presence of a "T" at position 31 indicates that the individual will have reduced symptomology associated with a neuropsychiatric disorder.

In another embodiment, the presence of an "A" at position 31, as numbered in SEQ ID NO: 1 indicates that a person will have an increased likelihood of being diagnosed with a neuropsychiatric disorder as compared with an individual having a "T" at the position.

The invention also relates to a kit for determining the genotype of a nucleotide corresponding to position 31 of SEQ ID NO: 1 in a polynucleotide sequence of interest. The kit comprises one or more nucleic acid probes, wherein one of said probes hybridizes to the polynucleotide sequence of interest, wherein the polynucleotide sequence of interest comprises the BDNF gene, its complement, or portion thereof and wherein the polynucleotide sequence of interest includes a nucleotide corresponding to position 31 of SEQ ID NO: 1. The kit can also comprise control nucleic acid samples representing the genotype of at least one of the group consisting of; an individual homozygous for an "A" at nucleotide position 31 of a BDNF gene, an individual homozygous for a "T" at nucleotide position 31 of a BDNF gene, and an individual heterozygous for said position, wherein position 31 corresponds to position 31 of SEQ ID NO: 1. The kits of the present invention are particularly suited for use in the method of the present invention, e.g., for predicting the likelihood that an individual will have or be diagnosed with a neuropsychiatric disorder, such as a bipolar disorder. In one embodiment, the kit comprises an SBE-FRET primer, wherein said primer hybridizes to a polynucleotide sequence comprising position 31 of SEQ ID NO: 1. In one embodiment, the polynucleotide sequence of interest is at least about 10 nueleotides in length. In another embodiment, the polynucleotide sequence is at least about 20 nueleotides in length.

In still another embodiment, the invention relates to a microarray, wherein the microarray has immobilized thereon a plurality of probes, wherein at least one of said probes is specific for the variant form of the single nucleotide polymorphism at position 31 of SEQ ID NO: 1. In another embodiment, at least one of the probes is specific for the reference form of the single nucleotide polymorphism at position 31 of SEQ ID NO: 1.

The invention also relates to a nucleic acid molecule, wherein the nucleic acid molecule comprises a nucleic acid sequcnce which is at least 10 nucleotides in length. Said nucleic acid molecule includes a nucleotide corresponding to position 31 of SEQ ID NO: 1 or its complement wherein said nucleotide at position 31 of SEQ ID NO: 1 is an "A."

### BRIEF DESCRIPTION OF THE DRAWINGS

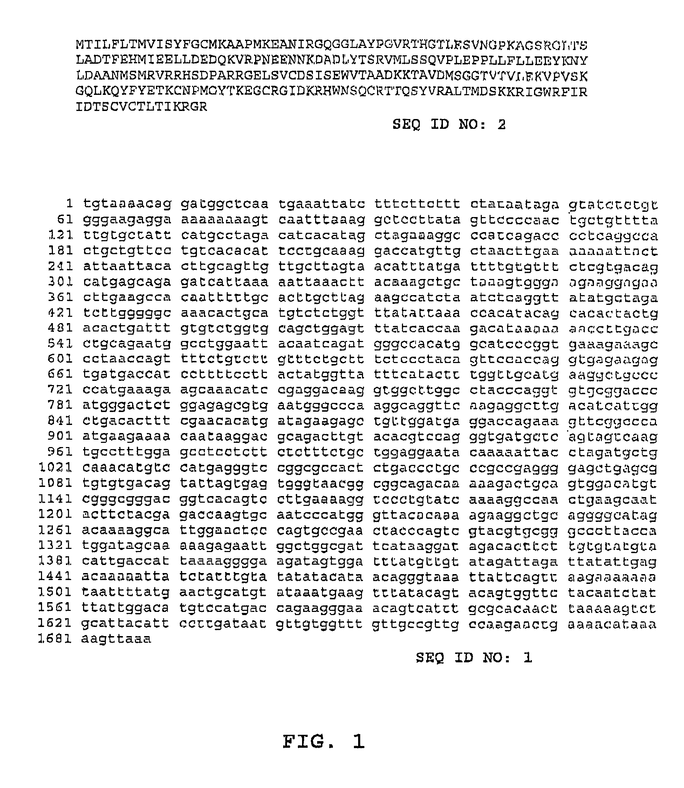

Figure 1 shows the polypeptide and polynucleotide sequence of BDNF, GenBank Accession M61181, SEQ ID NOs: 2 and 1, respectively.
Figure 2 shows the estimated relative risk of developing bipolar disorder based on data and analyses from the indicated groups of affected individuals and described in the Exemplification.

### DETAILED DESCRIPTION OF THE INVENTION

The development and maintenance of the vertebrate nervous system depends, in part, on the physiological availability of neuronal survival proteins known as neurotrophic factors. Neurotrophic factors play a role in maintaining neurons and their differentiated phenotypes in the adult nervous system. Nerve growth factor (NGF) remains the best characterized neurotrophic factor. However, brain-derived neurotrophic factor (BDNF) has been cloned and shown to be homologous to NGF (Leibrock *et al., Nature 341*:149-152 (1989); Hofer *et al*., *EMBO J. 9*:2459-2464 (1990); Maisonpierre *et al., Genomics 10*:558-568 (1991)). BDNF is initially synthesized as a 247 amino acid protein precursor that is subsequently cleaved to yield the mature protein. The mature form of BDNF essentially corresponds to the C-terminal half of its precursor and comprises 119 amino acids. In the developing rat, BDNF expression undergoes an increase from initially low levels, and in the adult rat central nervous system, BDNF is expressed at its highest level in the hippocampus. Expression of BDNF is detectable in adult tissues outside of the central nervous system only in heart, lung and skeletal musele (Maisonpierre *et al., Science*, 247:1446-1451 (1990); Hofer *et al., EMBO J.*, 9:2459-2464 (1990)).

As used herein, polymorphism refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 10% or 20% of a selected population. A polymorphic locus may be as small as one base pair, in which case it is referred to as a single nucleotide polymorphism.

As described herein, polymorphisms in the gene for BDNF have been discovered. In one embodiment, a single polymorphism from T to A at nucleotide position 31 in the BDNF gene, as numbered in SEQ ID NO: 1, or at a nucleotide position corresponding thereto, has been discovered. It has also been discovered that one or more single nucleotide polymorphisms within the nucleotide sequence encoding the amino acid prepro portion of the BDNF gene product arc correlated with a reduced incidence of bipolar disorder in the sample population assessed as described herein. For example, a single polymorphism from G to A at nucleotide position 858 of SEQ ID NO: 1, resulting in an amino acid change from valine to methionine at amino acid position -63 (relative to the start of the mature protein), or at an amino acid position corresponding thereto, is correlated with a reduced incidence of bipolar disorder in the sample population assessed as described herein. This polymorphism resides within the amino acid precursor portion (the prepro portion) which is cleaved from the mature protein.

It appears that the variant allele at position 858 of BDNF may contribute to protection or reduction in symptomology with respect to bipolar disorder. Alternatively, this particular polymorphism maybe one of group of two or more polymorphisms in the BDNF gene which contributes to the presence, absence or severity of the neuropsychiatric disorder, e.g., bipolar disorder. Therefore, because of the linkage disequilibrium, the transmission of at "T" at position 31 as numbered in SEQ ID NO: 1 is linked to transmission of "A" at position 858.

Variation at nucleotide 31 of SEQ ID NO: 1 is not in the coding region of the BDNF gcnc. Therefore, in terms of a phcnotypic effect on the BDNF protein, variation at position 31 is silent. However, because blocks of the genome are consistently inherited together in a population (linkage disequilibrium), nearby linked SNPs (whether silent or not) may also reveal an association to an underlying causative SNP. As described herein, the presence of "T" at position 31 of the BDNF gene (as numbered in SEQ ID NO: 1) is in complete linkage disequilibrium with a variation at position 858, Position 858, in turn, shows a variation from random in the transmission ofthe reference "G" and variant "A" alleles from an individual parent who is heterozygous for the BDNF alleles to an offspring diagnosed with bipolar disorder. As described herein, the transmission of "A" at position 858 is associated with a reduced incidence of bipolar disorder. Therefore, because of the linkage disequilibrium, the transmission of "T" at position 31 as numbered in SEQ ID NO: 1 is linked to transmission of "A" at position 858.

Therefore, while not wishing to be bound by theory, variation at position 31 can aid in the diagnosis or prognosis of a neuropsychiatric disorder, such as bipolar disorder, due to linkage disequilibrium of position 31 with position 858. Determination of the identity of nucleotide 31 of the BDNF gene, as numbered in SEQ ID NO: 1, can be used to aid in the formulation of a diagnosis or prognosis of neuropsychiatric disease, such as bipolar disorder. Furthermore, as more allelic variations arc discovered in genes involved in neuropsychiatric disorders, the ability to assess the genotype of an individual at one or more loci could facilitate the formulation of a diagnosis or prognosis of neuropsychiatric diseases, such as bipolar disorder.

Thus, the invention relates to a method for predicting the likelihood that an individual will have a neuropsychiatric disorder, or for aiding in the diagnosis of a neuropsychiatric disorder, e.g., bipolar disorder, or a greater likelihood of having reduced symptomology associated with a neuropsychiatric disorder, e.g., bipolar disorder, comprising the steps of obtaining a DNA sample from an individual to be assessed and determining the nucleotide genotype at nucleotide position 31 of the BDNF gene as numbered in SEQ ID NO: 1. In one embodiment, the nucleotide present at position 31 is identified. In a preferred embodiment, the neuropsychiatric disorder is bipolar disorder. In a particular embodiment, the individual is an individual at risk for development of bipolar disorder. In another embodiment the individual exhibits clinical symptomology associated with bipolar disorder. In one embodiment, the individual has been clinically diagnosed as having bipolar disorder.

As used herein, "position 31" and "position 858 " refer to nucleotide positions of the BDNF gene corresponding to positions 31 and 858, respectively of SEQ ID NO: 1, or the complement thereof. When referring to the complementary strand, it is understood that the complementary base of the indicated nucleotide of interest. The nucleotide positions of the polymorphisms can be referred to in a number of different ways. For convenience, the following table provides a cross-reference between two common numbering schemes: the numbering based on GenBank sequence M61181 (SEQ ID NO : 1), and numbering based on the starting codon of the prepro protein (where the first nucleotide in the starting ATG codon is "1" and for example, the nucleotide upstream of "1" is "-1."

**TABLE I**

| Nucleotide Position of SEQ ID NO: 1 | Nucleotide Position Relative to First Position of Starting ATG¹ |
|---|---|
| 31 | -633 |
| 858 | 196 |

| | |
|---|---|
| 1. Position 663 of SEQ ID NO: 1. | |

The genetic material to be assessed can be obtained from any nucleated cell from the individual. For assay of genomic DNA, virtually any biological sample (other than pure red blood cells) is suitable. For example, convenient tissue samples include whole blood, somen, saliva, tears, urine, fecal material, sweat, skin and hair. For assay of cDNA or mRNA, the tissue sample must be obtained from an organ in which the target nueleie acid is expressed. For example, cells from the central nervous system (such as cells of the hippocampus), neural crest-derived cells, skin, heart, lung and skeletal muscle are suitable sources for obtaining cDNA for the BDNF gene. Neural crest derived cells include, for example, melanocytes and keratinocytes.

Many of the methods described herein require amplification of DNA from target samples. This can be accomplished by e.g., PCR. *See generally PCR Technology: Principles* *and Applications for DNA Amplification* (ed. H.A. Erlich, Freeman Press, NY, NY, 1992); *PCR Protocols: A Guide to Methods and Applications* (eds. Innis, *et al*., Academic Press, San Diego, CA, 1990); Mattila *et al., Nucleic Acids Res.* 19, 4967 (1991); Eckert *et al., PCR Methods and Applications* 1, 17(1991); *PCR* (eds. McPherson *et al*., IRL Press, Oxford); and U.S. Patent 4,683,202.

Other suitable amplification methods include the ligase chain reaction (LCR) (see Wu and Wallace, *Genomics* 4, 560 (1989), Landegren *et al., Science* 241, 1077(1988), transcription amplification (Kwoh *et al., Proc. Natl. Acad. Sci. USA* 86, 1173 (1989)), and self-sustained sequence replication (Guatelli *et al., Proc. Nat. Acad. Sci. USA*, 87, 1874 (1990)) and nucleic acid based sequence amplification (NASBA). The latter two amplification methods involve isothermal reactions based on isothermal transcription, which produce both single stranded RNA (ssRNA) and double stranded DNA (dsDNA) as the amplification products in a ratio of about 30 or 100 to 1, respectively.

The nucleotide which occupies the polymorphic site of interest (e.g., nucleotide position 31 in BDNF as numbered in SEQ ID NO: 1) can be identified by a variety of methods, such as Southern analysis of genomic DNA; direct mutation analysis by restriction enzyme digestion; Northern analysis of RNA; denaturing high pressure liquid chromatography (DHPLC); gene isolation and sequencing; hybridization of an allele-specific oligonucleotide with amplified gene products; single base extension (SBE); or analysis of the BDNF protein. In a preferred embodiment, determination of the allelic form of BDNF is carried out using SBE-FRET methods as described in the examples, or using chip-based oligonucleotide arrays. A sampling of suitable procedures are discussed below in turn.

### 1. Allele-Specific Probes

The design and use of allele-specific probes for analyzing polymorphisms is described by e.g., Saiki *et al., Nature* 324, 163-166 (1986); Dattagupta, EP 235,726, Saiki, WO 89/11548. Allele-specific probes can be designed that hybridize to a segment of target DNA from one individual but do not hybridize to the corresponding segment from another individual due to the presence of different polymorphic forms in the respective segments from the two individuals. Hybridization conditions should be sufficiently stringent that there is a significant difference in hybridization intensity between alleles, and preferably an essentially binary response, whereby a probe hybridizes to only one of the alleles. Hybridizations are usually performed under stringent conditions, for example, at a salt concentration of no more than 1 M and a temperature of at least 25°C. For example, conditions of 5X SSPE (750 mM NaCl, 50 mM NaPhosphate, 5 mM EDTA, pH 7.4) and a temperature of 25-30°C, or equivalent conditions, are suitable for allele-specific probe hybridizations. Equivalent, conditions can be determined by varying one or more of the parameters given as an example, as known in the art, while maintaining a similar degree of identity or similarity between the target nucleotide sequence and the primer or probe used.

Some probes are designed to hybridize to a segment of target DNA such that the polymorphic site aligns with a central position (e.g., in a 15-mer at the 7 position; in a 16-mer, at either the 8 or 9 position) of the probe. This design of probe achieves good discrimination in hybridization between different allelie forms.

Allele-specific probes are often used in pairs, one member of a pair showing a perfect match to a reference form of a target sequence and the other member showing a perfect match to a variant form. Several pairs of probes can then be immobilized on the same support for simultaneous analysis of multiple polymorphisms within the same target sequence. Allele specific probes can comprise DNA, peptide nucleic acid (PNA) and RNA, or combinations thereof.

### 2. Tiling Arrays

The polymorphisms can also be identified by hybridization to nucleic acid arrays, some examples of which are described in WO 95/11995. WO 95/11995 also describes subarrays that arc optimized for detection of a variant form of a precharacterized polymorphism. Such a subarray contains probes designed to be complementary to a second reference sequence, which is an allclic variant of the first reference sequence. The second group of probes is designed by the same principles, except that the probes exhibit complementarity to the second reference sequence. The inclusion of a second group (or further groups) can be particularly useful for analyzing short subsequences of the primary reference sequence in which multiple mutations are expected to occur within a short distance commensurate with the length of the probes (e.g., two or more mutations within 9 to 21 bases).

### 3. Allele-Specific Primers

An allele-specific primer hybridizes to a site on target DNA overlapping a polymorphism and only primes amplification of an allelic form to which the primer exhibits perfect complementarity. See Gibbs, *Nucleic Acid Res.* 17, 2427-2448 (1989), This primer is used in conjunction with a second primer which hybridizes at a distal site. Amplification proceeds from the two primers, resulting in a detectable product which indicates the particular allelic form is present. A control is usually performed with a second pair of primers, one of which shows a single base mismatch at the polymorphic site and the other of which exhibits perfect complementarity to a distal site. The single-base mismatch prevents amplification and no detectable product is formed. The method works best when the mismatch is included in the 3'-most position of the oligonucleotide aligned with the polymorphism because this position is most destabilizing to elongation from the primer (see, e.g., WO 93/22456).

### 4. Direct-Sequencing

The direct analysis of the sequence of polymorphisms of the present invention can be accomplished using either the dideoxy chain termination method or the Maxam Gilbert method (see Sambrook *et al., Molecular Cloning, A Laboratory Manual* (2nd Ed., CSHP, New York 1989); Zyskind *et al., Recombinant DNA Laboratory Manual*, (Acad. Press, 1988)).

### 5. Denaturing Gradient Gel Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution. Erlich, ed., *PCR Technology, Principles and Applications for DNA Amplification*, (W.H. Freeman and Co, New York, 1992), Chapter 7.

### 6. Single-Strand Conformation Polymorphism Analysis

Alleles of target sequences can be differentiated using single-strand conformation polymorphism analysis, which identifies base differences by alteration in electrophoretic migration of single stranded PCR products, as described in Orita *et al., Proc. Nat. Acad. Sci.* 86, 2766-2770(1989). Amplified PCR products can be generated as described above, and heated or otherwise denatured, to form single stranded amplification products. Single-stranded nucleic acids may refold or form secondary structures which are partially dependent on the base sequence. The different electrophoretie mobilities of single-stranded amplification products can be related to base-sequence differences between alleles of target sequences.

### 7. Single-Base Extension

An alternative method for identifying and analyzing polymorphisms is based on single-base extension (SBE) of a fluorescently-labeled primer coupled with fluorescence resonance energy transfer (FRET) between the label of the added base and the label of the primer. Typically, the method, such as that described by Chen *et al.,* (*PNAS 94*:10756-61 (1997), incorporated herein by reference) uses a locus-specific oligonueleotide primer labeled on the 5' terminus with 5-carboxyfluorescein (FAM). This labeled primer is designed so that the 3' end is immediately adjacent to the polymorphic site of interest. The labeled primer is hybridized to the locus, and single base extension of the labeled primer is performed with fluorescently labeled dideoxyribonucleotides (ddNTPs) in dye-terminator sequencing fashion, except that no deoxyribonuclcotides are present. An increase in fluorescence of the added ddNTP in response to excitation at the wavelength of the labeled primer is used to infer the identity of the added nucleotide.

The polymorphisms of the invention may contribute to the protection of an individual against bipolar disorder in different ways. The polymorphisms may contribute to phenotype by affecting protein structure. By altering amino acid sequence, the polymorphism may alter the function of the encoded protein. The polymorphisms may exert phenotypic effects indirectly via influence on replication, transcription, and translation. For example, the substitution of a methionine for a valine in the prepro portion of the BDNF gene product may create an alternative translation start site which alters the length of the gene product and the prepro portion itself. Alteration of the length of the gene product may affect cleavage of the mature protein either positively or negatively. Alternatively, the presence of the variant amino acid may alter the properties of the gene product so as to alter cleavage of the gene product. More than one phenotypic trait may be affected. For example, other neuropsychiatric disorders which are believed to be alternate expressions of a bipolar genotype, including variants of schizoaffective disorder, recurrent unipolar depression and hypomania (bipolar II disorder), may also be affected by the BDNF polymorphisms described herein. Additionally, the described polymorphisms may predispose an individual to a distinet mutation that is causally related to a certain phenotype, such as susceptibility or resistance to bipolar disorder. The discovery of the polymorphisms and correlation with bipolar disorder facilitates biochemical analysis of the variant and the development of assays to characterize the variant and to screen for pharmaceuticals that interact directly with one or another form of the protein.

Alternatively, the polymorphisms may be one of a group of two or more polymorphisms in the BDNF gene which contributes to the presence, absence or severity of the neuropsychiatric disorder, e.g., bipolar disorder. An assessment of other polymorphisms within the BDNF gene can be undertaken, and the separate and combined effects of these polymorphisms on the neuropsychiatric disorder phenotype can be assessed.

Correlation between a particular phenotype, e.g., the bipolar phenotype, and the presence or absence of a particular allcle is performed for a population of individuals who have been tested for the presence or absence of the phenotype. Correlation can be performed by standard statistical methods such as a Chi-squared test and statistically significant correlations between polymorphic form(s) and phenotypic characteristics are noted, For example, as described herein, it has been found that the presence of the BDNF variant allele, having an A at polymorphic site 858 (as numbered in SEQ ID NO: 1), correlates negatively with bipolar disorder with a p value of p=0.004 by Chi-squared test.

This correlation can be exploited in several ways. In the case of a strong correlation between a particular polymorphic form, e.g., the reference allele for BDNF, and a disease for which treatment is available, e.g., bipolar disorder, detection of the polymorphic form in an individual may justify immediate administration of treatment, or at least the institution of regular monitoring of the individual. Detection of a polymorphic form correlated with a disorder in a couple contemplating a family may also be valuable to the couple in their reproductive decisions. For example, the female partner might elect to undergo *in vitro* fertilization to avoid the possibility of transmitting such a polymorphism from her husband to her offspring. In the case of a weaker, but still statistically significant correlation between a polymorphic form and a particular disorder, immediate therapeutic intervention or monitoring may not be justified. Nevertheless, the individual can be motivated to begin simple life-style changes (e.g., therapy or counseling) that can be accomplished at little cost to the individual but confer potential benefits in reducing the risk of conditions to which the individual may have increased susceptibility by virtue of the particular allele. Furthermore, identification of a polymorphic form correlated with enhanced receptiveness to one of several treatment regimes for a disorder indicates that this treatment regime should be followed for the individual in question.

Furthermore, it may be possible to identify a physical linkage between a genetic locus associated with a trait of interest (e.g., bipolar disorder) and polymorphic markers that are not associated with the trait, but are in physical proximity with the genetic locus responsible for the trait and co-segregate with it. Such analysis is useful for mapping a genetic locus associated with a phenotypic trait to a chromosomal position, and thereby cloning gene(s) responsible for the trait. *See* Lander *et al., Proc. Natl. Acad. Sci. (USA)* 83, 7353-7357 (1986); Lander *et al., Proc. Natl. Acad. Sci. (USA)* 84, 2363-2367 (1987); Donis-Keller *et al., Cell* 51, 319-337 (1987); Lander *et al*., *Genetics* 121, 185-199 (1989)). Genes localized by linkage can be cloned by a process known as directional cloning. See Wainwright, *Med. J. Australia* 159, 170-174(1993); Collins, *Nature Genetics* 1, 3-6 (1992).

Linkage studies arc typically performed on members of a family. Available members of the family are characterized for the presence or absence of a phenotypic trait and for a set of polymorphic markers. The distribution of polymorphic markers in an informative meiosis is then analyzed to determine which polymorphic markers co-segregate with a phenotypic trait. *See*, *e.g.*, Kerem *et al., Science* 245, 1073-1080 (1989); Monaco *et al., Nature* 316, 842 (1985); Yamoka *et al., Neurology* 40, 222-226 (1990); Rossiter *et al., FASEB Journal* 5, 21-27 (1991).

Linkage is analyzed by calculation of LOD (log of the odds) values. A LOD value is the relative likelihood of obtaining observed segregation data for a marker and a genetic locus when the two are located at a recombination fraction 0, versus the situation in which the two are not linked, and thus segregating independently (Thompson & Thompson, *Genetics in Medicine* (5th ed, W.B. Saunders Company, Philadelphia, 1991); Strachan, "Mapping the human genome" in *The Human Genome* (BIOS Scientific Publishers Ltd, Oxford), Chapter 4). A series of likelihood ratios are calculated at various recombination fractions (0), ranging from θ = 0.0 (coincident loci) to θ = 0.50 (unlinked). Thus, the likelihood at a given value of 0 is: probability of data if loci linked at θ to probability of data if loci unlinked. The computed likelihoods are usually expressed as the log₁₀ of this ratio (i.e., a LOD score). For example, a LOD score of 3 indicates 1000:1 odds against an apparent observed linkage being a coincidence. The use of logarithms allows data collected from different families to be combined by simple addition. Computer programs are available for the calculation of LOD scores for differing values of θ (e.g., LIPED, MLINK (Lathrop, *Proc. Nat. Acad. Sci. (USA)* 81, 3443-3446 (1984)). For any particular LOD score, a recombination fraction may be determined from mathematical tables. *See* Smith *et al., Mathematical tables for research workers in human genetics* (Churchill, London, 1961); Smith, *Ann*. *Hum. Genet*. 32, 127-150 (1968). The value of θ at which the LOD score is the highest is considered to be the best estimate of the recombination fraction.

Positive LOD score values suggest that the two loci arc linked, whereas negative values suggest that linkage is less likely (at that value of θ) than the possibility that the two loci are unlinked. By convention, a combined LOD score of+3 or greater (equivalent lo greater than 1000:1 odds in favor of linkage) is considered definitive evidence that two loci are linked. Similarly, by convention, a negative LOD score of -2 or less is taken as definitive evidence against linkage of the two loci being compared. Negative linkage data are useful in excluding a chromosome or a segment thereof from consideration. The search foeuses on the remaining non-excluded chromosomal locations.

The invention also encompasses kits for detecting the presence of proteins or nucleic acid molecules of the invention in a biological sample. For example, the kit can comprise a compound or agent (e.g., one or more nucleic acid probes) cahable of detecting protein or mRNA (or cDNA produced from the mRNA) in a biological sample or means for determining the identity of a a particular nucleotide or amino acid of the BDNF gene or protein, respectively. For example, in one embodiment, the kit comprises a means for determining the identity of nucleotide 31 of BDNF gene as numbered in SEQ ID NO: 1. In another embodiment, the compound or agent, such as oligonucleotide(s) or antibody(ics) is labeled. In still another embodiment, the kit includes fluorescently labeled dideoxynucleotides. The kit can also comprise control samples for use as standards, representing individuals homozygous for the reference or variant nucleotide in the case of analyzing nucleic acid, or the reference or variant amino acid in the case of analyzing proteins, or representing a heterozygous individual. The compound or agent can be packaged in a suitable container. The kit can further comprise instructions for using the kit to detect protein or nucleic acid.

The invention further pertains to compositions, e.g., vectors, comprising a nuclcotide sequence encoding variant BDNF gene. In one embodiment, the gene comprises BDNF sequences including position 31 of SEQ ID NO: 1.

For example, the BDNF gene or variants thereof can be expressed in an expression vector in which a variant gene is operably linked to a native or other promoter. Usually, the promoter is a cukaryotic promoter for expression in a mammalian cell. The transcription regulation sequences typically include a heterologous promoter and optionally an enhancer which is recognized by the host. The selection of an appropriate promoter, for example trp, lac, phage promoters, glycolytic enzyme promoters and tRNA promoters, depends on the host selected. Commercially available expression vectors can be used. Vectors can include host-recognized replication systems, amplifiable genes, selectable markers, host sequences useful for insertion into the host genome, and the like.

The means of introducing the expression construct into a host cell varies depending upon the particular construction and the target host. Suitable means include fusion, conjugation, transfection, transduction, electroporation or injection, as described in Sambrook, *supra.* A wide variety of host cells can be employed for cxprcssion of the variant gene, both prokaryotic and eukaryotic. Suitable host cells include bacteria such as *E. coli*, yeast, filamentous fungi, insect cells, mammalian cells, typically immortalized, *e.g.*, mouse, CHO, human and monkey cell lines and derivatives thereof. Preferred host cells are able to process the variant gene product to produce an appropriate mature polypeptide. Processing includes glycosylation, ubiquitination, disulfide bond formation, general post-translational modification, and the like.

It is also contemplated that cells can be engineered to express the BDNF allele of the invention by gene therapy methods. For example, DNA encoding the BDNF gene product, or an active fragment or derivative thereof, can be introduced into an expression vector, such as a viral vector, and the vector can be introduced into appropriate cells in an animal. In such a method, the cell population can be engineered to inducibly or constitutively express active BDNF gene product. Tn a preferred embodiment, the vector is delivered to the bone marrow, for example as described in Corey *et al.* (*Science* 244:1275-1281 (1989)).

The invention further provides transgenic nonhuman animals capable of expressing an exogenous BDNF gene and/or having one or both alleles of an endogenous BDNF gene inactivated. Expression of an exogenous gene is usually achieved by operably linking the gene to a promoter and optionally an enhancer, and microinjecting the construct into a zygole. *See* Hogan *et al.*, "Manipulating the Mouse Embryo, A Laboratory Manual," Cold Spring Harbor Laboratory. Inactivation of endogenous genes can be achieved by forming a transgene in which a cloned variant gene is inactivated by insertion of a positive selection marker. *See* Capecchi, *Science* 244, 1288-1292 (1989), The transgene is then introduced into an embryonic stem cell, where it undergoes homologous recombination with an endogenous gene. Mice and other rodents are preferred animals. Such animals provide useful drug screening systems.

The invention further relates to an oligonucleotide microarray having immobilized thereon a plurality of oligonucleotide probes wherein at least one of said probes is specifie for the nucleotide at position 31 of SEQ ID NO: 1. In one embodiment, the invention relates to an oligonucleotide microarray having immobilized thereon a plurality of oligonucleotide probes wherein at least one of said probes is specific for the variant nuclcolide at position 31 of SEQ ID NO: 1. In another embodiment, the invention relates to an oligonueleotide microarray having immobilized thereon a plurality of oligonucleotide probes wherein at least one of said probes is specific for the reference nucleotide at position 31 of SEQ ID NO: 1. The nucleic acid sequence surrounding the position 31 of SEQ ID NO; 1 can be used to design suitable oligonucleotide probes, and the preparation of such oligonucleotide microarrays is well known in the art.

The invention will be further illustrated by the following non-limiting examples. The teachings of the references cited herein are incorporated herein by reference in their entirety.

### EXAMPLES

### Sample Population

A sample population oF 150 trios was initially assessed by genotyping methods for heterozygousity with respect to the BDNF reference and variant alleles as described herein. A trio included two parents and an offspring diagnosed as having bipolar disorder according to the American Psychiatric Association's Diagnostic and Statistical Manual of Mental Disorders. Of the 150 trios assessed, 98 of these trios had at least one parent who was heterozygous for the BDNF reference and variant alleles; these 98 trios were selected for further study, as the heterozygousity of the parent allowed a determination of which allele the parent transmitted to the bipolar offspring. The bipolar offspring in the trios were assessed by genotyping methods to determine which BDNF allele had been transmitted to them by the heterozygous parent. In instances where two parents had two offspring diagnosed with bipolar disorder, each trio (i.e., two parents and one offspring) was considered individually.

### SBE-FRET Protocol

The genotyping method used for these studies was based on single-base extension (SBE) and fluorescence resonance energy transfer (FRET). A locus-specific primer (FRET primer; 5'-GGCTGACACTTTCGAACAC (SEQ ID NO: 3) was ordered 5'labeled with FAM. The primer was designed so that the 3' end hybridized immediately adjacent to the polymorphic site of interest (e.g., nucleotide 858), such that a single-base extension of the primer would result in the addition of a nucleotide complementary to the template polymorphic site of interest. Locus specific primer for other positions of interest, such as position 31 of SEQ ID NO: 1, can be readily designed and labeled with FAM using methods well known in the art. The locus of interest was amplified and single base extension of the FRET primer was performed with fluorescently labeled ddNTPs in dye-terminator sequencing fashion, except that no deoxyribonucleotides arc present. PCR primers were:

Forward PCR primer 5'-TGTAAAACGACGGCCAGTCTTGACATCA
TTGGCTGACACT (SEQ ID NO: 4); and

Reverse PCR primer 5'-TAATACGACTCACTATAGGGGTACAAGT
CTGCGTCCTTATTGTTT (SEQ ID NO: 5).

The ddNTP corresponding to the variant base (A) was labeled with TAMRA, and the reference base (G) was labeled with ROX. Depending on the genotype of the individual, the FRET primer was extended with a ROX-labeled or TAMRA-labeled ddNTP. Upon incorporation of either ROX- or TAMRA-labeled ddNTPs, energy transfer occurs between the donor dye (FAM on FRET primer) and the acceptor dye (the ROX- or TAMRA-labeled ddNTP). An increase in the fluorescence intensity of one (for a homozygote) or both (for a heterozygote) of the acceptor dyes was used to infer the genotype of an individual.

Summary of experimental procedures used in the above-described analysis.
I Amplify locus of interest
II Clean-up of PCR products with shrimp alkaline phosphate (SAP) and Exonuclease 1 (EXO)
III Single-base extension/fluorescence detection in ABI7700

### I Amplification of locus of interest - for 96-well plate

**TABLE II:**

| **PCR MIX** | | |
|---|---|---|
| | Each Reaction (µL) | For 96-well plage (µL) |
| 10 mM dNTP | 0.05 | 5.2 |
| 10X PCRII buffer | 2.0 | 208 |
| 25 mM MgCl2 | 1.2 | 125 |
| 20 µM PCR primer F | 0.25 | 26 |
| 20 µM PCR primer R | 0.25 | 26 |
| ddH₂O | 11.05 | 1149 |
| 5 U/µL Amplitaq-gold | 0.2 | 20.8 |
| | 15 | |

Fifteen microliters of the PCR mix were added to a 96-well MJ plate. Five microliters of genomic DNA (5 ng/µL) were added to the aliquoted PCR mix. (5 µL of 1 ng/µL is often adequate).

The plate was sealed with MJ plate-seal 'A'.

PCK was conducted using the following program:
96°C x 10 minutes
96°C x 30 seconds, 50C x 1 minute, 72C x 1 minute for 35 cycles
72°C x 10 minutes followed by a hold at 4°C

### II PCR product clean-up

**TABLE III:**

| **SAP/EXO MIX** | | |
|---|---|---|
| | Each reaction (µL) | For 96-well plate (µL) |
| Shrimp alkaline phosphatase (1U/µL) | 1.0 | 104 |
| Exonuclease 1 (10 U/µL) | 0.05 | 5.2 |
| 10X SAP buffer | 1.0 | 104 |
| ddH₂O | 2.95 | 306.8 |
| | 5.0 | |

Five microliters of SAP/EXO mix were added to a clean MJ plate. Five microliters of the PCR product were added directly to the aliquoted SAP/EXP mix. The PCR plates were spun down and sealed with Microseal A film. The mixture was incubated at 37°C for 45 minutes and then at 96°C for 15 minutes.

### III Single-base extension/fluorescence detection in ABI7700

### (The reactions were carried out in the same MJ plate used for SAP/EXO step, capped with 8-strip MicroAmp optical caps)

The ddNTPs that should be incorporated in the genotyping reaction were selected. In this experiment, TAMRA was used to identify the variant base and ROX for the reference base, although other possibilities exist.

**TABLE IV:**

| **SBE-FRET MIX** | | |
|---|---|---|
| | Each reaction (µL) | For 96-well plate (µL) |
| FAM primer (100 uM) | 0.02 | 2.08 |
| ROX ddNTP (100 um) | 0.02 | 2.08 |
| TAMRA ddNTP (100 um) | 0.02 | 2.08 |
| Thermoseq. Buffer (10x) | 2.0 | 208 |
| ddH₂O | 7.9 | 821.6 |
| Thermosequenase (32 U/µL) | 0.016 | 1.7 |
| | 10.0 | |

- Ten microliters of SBE-FRET mix were added to the MJ plates containing 10 µL SAP/Exo treated PCR products.
- The plates were tapped on bench to mix, they can also be spun briefly if necessary.
- The wells were capped with optical caps. The capped wells can be rolled with roller if necessary.
- The plates were placed in a thermocycling detector apparatus (AB17700).
- The plates were incubated for 6 cycles of (for a 20 µL reaction) as follows:
   96°C x 15 seconds
   50°C x 30 seconds
   60°C x 30 seconds

Data were collected in the 60°C stage using detection settings suitable for measuring TAMRA and ROX fluorescence.

Data were analyzed by plotting ROX fluorescence versus TAMRA fluorescence and comparing the values between samples, control samples containing no template and samples of known geneotype. Typically, homozygous reference controls have little or no TAMRA fluorescence, homozygous variant controls have little or no ROX fluorescence and heterozygous controls have similar TAMRA and ROX fluorescence.

### Results

Data from the work described herein has shown that there is a variation from random (i.e., that which would be expected by chance) in the transmission at position 858 of SEQ ID NO: 1 of the reference (G) and variant (A) alleles from an individual parent who is heterozygous for the BDNF alleles to an offspring diagnosed with bipolar disorder.

The data demonstrated that the variant allele (A) is transmitted less frequently (34 of 98 times) to the bipolar offspring than would be expected by chance, while the reference allele (G) is transmitted more frequently (64 of 98 times) than would be expected by chance (p= 0.004), In the general population (in which about 0.8 percent of the individuals are diagnosed with bipolar disorder), the variant (A) allele occurs with a frequency of 15 percent, while the reference allele occurs with a frequency of 85 percent. In the sample population assessed as described herein, in which all of the individuals are diagnosed with bipolar disorder, the variant allele occurs with a frequency of 7 percent. Thus, it appears that the variant allcle may contribute to protection or reduction in symptomology with respect to bipolar disorder.

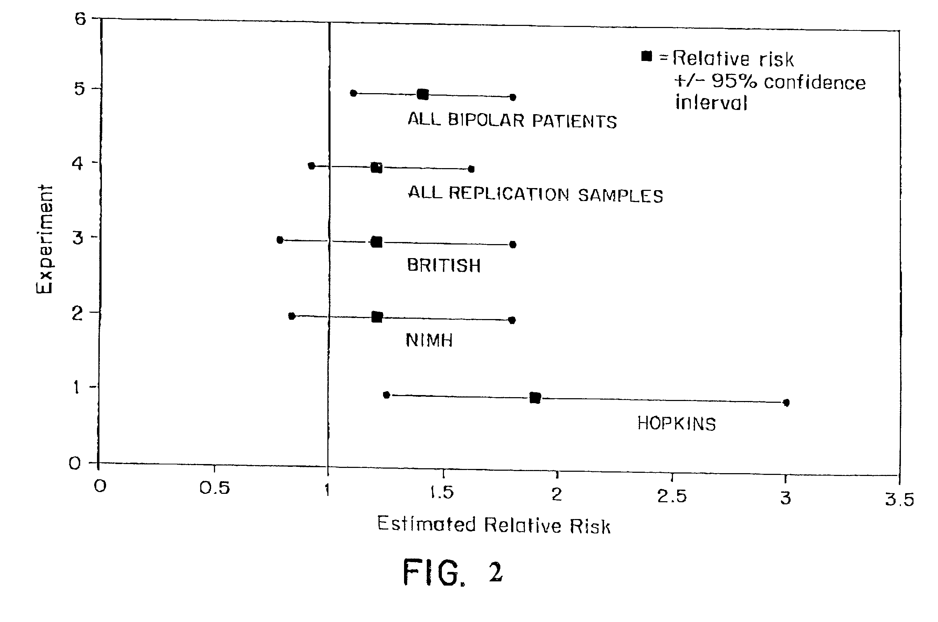

Figure 2 and Table V show data obtained from additional human samples.

**TABLE V**

| Samples | Trans | Untrans. | p val | # trans | relative risk | 95% CF | # trios |
|---|---|---|---|---|---|---|---|
| Hopkins | 55 | 29 | 0.0023 | 84 | 1.90 | 1.25-3.0 | 127 |
| U01 + NIMH | 50 | 42 | 0.2021 | 92 | 1.19 | 0.83-1.8 | 155 |
| British | 38 | 32 | 0.2366 | 70 | 1.19 | 0.77-1.8 | 145 |
| all repl. | 88 | 74 | 0.1357 | 162 | 1.19 | 0.91-1.8 | 300 |
| all BP | 143 | 103 | 0.0054 | 246 | 1.39 | 1.1-1.8 | 427 |

"Hopkins" refers to a to a group of patients with bipolar disorder obtained in collaboration with Johns Hopkins. "U01 and NIMH" refer to a group of 155 trios, some of which are from Johns Hopkins and some of which are from the Genetics Initiative at the NIMH. "British" refers to 145 trios from 5 collaborators in England.

In Table V, "Trans" is the number of times the allele in question (at position 858, in this case the reference allele) was transmitted from a heterozygous parents to a bipolar child. "Untrans" is the number of times the other (variant) allele was passed from the heterozygous parent to the bipolar child. The number of trios used is show in the column labeled " 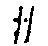 trios" and is the number of trios for which genotypes were available. Not all of the parents were considered to be "informative". To be included in the analysis, the parent in question had to be a heterozygote.

The relative risk (estimated relative risk on Figure 2) is defined as the transmission ratio in trios (i.e # transmitted alleles/# untransmitted alleles). Under a multiplicative disease model, this is an estimator of genotypic relative risk. The confidence interval was calculated using a binomial distribution.

The combination of "T" at position 31 and "A" at position 858 was tested and found to be in nearly complete linkage disequilibrium in both the Hopkins and the NIMH datasets.

While this invention has been particularly shown and described with references to preferred embodiments thereof, it will be understood by those skilled in the art that various changes in form and details may be made therein without departing from the scope of the invention encompassed by the appended claims.

## Claims

1. A kit for determining the genotype of an individual at a nucleotide corresponding to position 31 of a BDNF gene in a polynucleotide sequence of interest comprising:
a) one or more nucleic acid probes, wherein at least one of said probes hybridizes to the polynucleotide sequence, wherein the polynucleotide sequence comprises the BDNF gene, its complement, or portion thereof, and wherein the polynucleotide sequence includes a nucleotide corresponding to position 31 of SEQ ID NO 1; and
b) control nucleic acid samples representing the gene type of at least one of the group consisting of: an individual homozygous for a "A" at nucleotide position 31 of the BDNF gene, an individual homozygous for a "T" at nucleotide position 31 of the BDNF gene, and a individual heterozygous for said position, wherein position 31 corresponds to position 31 of SEQ ID NO 1.

2. A kit of claim 1, wherein:
a) the nucleic acid probe is an SBE-FRET primer, and wherein the SBE-FRET primer hybridizes to the polynucleotide sequence such that the nucleotide corresponding to position 31 of SEQ ID NO 1 is immediately adjacent to the 3' terminus of the SBE-FRET primer, and optionally wherein the SBE-FRET primer comprises SEQ ID NO 3; and/or further comprising fluorescently labeled didexynucleotides; and/or
b) the control nucleic acid samples comprise amplified DNA; and optionally wherein the control samples are amplified using primers comprising SEQ ID NO 4 and SEQ ID NO 5; and/or
c) the polynucleotide sequence of interest comprises a nucleic acid sequence of at least 10 nucleotides in length, wherein said nucleic acid of interest comprises a BDNF gene or portion thereof, including position 31 of SEQ ID NO 1; and optionally wherein the polynucleotide sequence of interest is at least 20 nucleotides in length.

3. A nucleic acid molecule comprising a nucleic acid sequence which is at least 10 nucleotides in length, wherein said nucleic acid molecule includes a nucleotide corresponding to position 31 of SEQ ID NO 1 or its complement, wherein said nucleotide at position 31 of SEQ ID NO 1 is an "A".

4. A nucleic acid molecule according to claim 3, wherein said nucleic acid sequence is at least 20 nucleotides in length.

5. A method for predicting the likelihood that an individual will be diagnosed with a bipolar disorder, comprising the steps of:
a) providing a DNA sample obtained from an individual to be assessed; and
b) determining the nucleotide present at nucleotide position 31 of brain-derived neurotrophic factor gene, as numbered in SEQ ID NO 1,
wherein the presence of a "T" at position 31 indicates that the individual has a reduced likelihood of being diagnosed with a bipolar disorder as compared with an individual having an "A" at that position.

6. A method according to claim 5, wherein the individual is an individual at risk for development of a bipolar disorder.

7. A method for predicting the likelihood that an individual will be diagnosed with a bipolar disorder, comprising the steps of:
a) providing a DNA sample obtained from an individual to be assessed; and
b) determining the nucleotide present at nucleotide position 31 of brain-derived neurotrophic factor gene, as numbered in SEQ ID NO 1,
wherein the presence of an "A" at position 31 indicates that the individual has an increased likelihood of being diagnosed with a bipolar disorder as compared with an individual having a "T" at that position.

8. A method according to claim 7, wherein the individual:
a) exhibits clinical symptoms of mania or mania and depression, or
b) is an individual at risk for development of a bipolar disorder.

9. A method according to any one of claims 5 to 8, wherein the nucleotide at position 31 is determined by single-base extension using a primer capable of hybridizing to SEQ ID NO 1, its complement or portions thereof, such that a nucleotide corresponding to nucleotide 31 of SEQ ID NO 1 is immediately adjacent to the 3' terminus of said primer; and optionally wherein the primer comprises SEQ ID NO 3 or its complement, and/or the primer is immobilized on a solid support.

10. An oligonucleotide microarray having immobilized thereon a plurality of probes, wherein at least one of said probes is specific for either the variant form or the reference form of the single nucleotide polymorphism at position 31 of SEQ ID NO 1.

11. A compound for use in the treatment of bipolar disorders in an individual, wherein the individual has had determined the nucleotide present at nucleotide position 31 of brain-derived neurotrophic factor gene as numbered is SEQ ID NO 1, and the compound is selected based upon said determination.
